# EUROPEAN PATENT SPECIFICATION

(11) **EP 1 713 392 B1**
(45) Date of publication and mention of the grant of the patent: **11.02.2009**
(21) Application number: 05702654.4
(22) Date of filing: 12.01.2005
(51) Int. Cl.: A61B 6/03, G01N 23/04, G06T 11/00

(54) **COMPUTED TOMOGRAPHY IMAGING WITH PIXEL STAGGERING AND FOCAL SPOT MODULATION**
COMPUTERTOMOGRAPHIE-DARSTELLUNG MIT PIXEL-VERSATZ UND FOKALER PUNKT-MODULATION
IMAGERIE PAR TOMODENSITOMETRIE A DECALAGE DE PIXELS ET MODULATION DE POINT FOCAL

(30) Priority: 29.01.2004 US 540208 P
(43) Date of publication of application: 25.10.2006
(73) Proprietor: Koninklijke Philips Electronics N.V., 5621 BA Eindhoven (NL)
(72) Inventor: HEUSCHER, Dominic, J., Cleveland, OH 44143 (US); UTRUP, Steven, J., Cleveland, OH 44143 (US); SHECHTER, Gilad, Cleveland, OH 44143 (US); KOEHLER, Thomas, Cleveland, OH 44143 (US)
(74) Representative: Volmer, Georg
(86) International application number: PCT/IB2005/050139
(87) International publication number: WO 2005/072612

(56) References cited:
- EP-A- 0 543 626
- DE-A- 10 164 233
- US-A- 4 754 468
- US-A- 4 817 123
- US-A- 5 510 622
- US-A1- 2002 176 530
- US-B1- 6 178 220
- US-B1- 6 256 369
- PATENT ABSTRACTS OF JAPAN vol. 1995, no. 06, 31 July 1995 (1995-07-31) & JP 07 084052 A (TOSHIBA CORP; others: 01), 31 March 1995 (1995-03-31)

## Description

The following relates to computed tomography imaging. It finds particular application in three-dimensional multi-slice and helical computed tomography imaging, and will be described with particular reference thereto. However, it also finds application in other imaging apparatuses and methods that employ arrays of x-ray detectors.

Computed tomography scanners employ arrays of x-ray detectors and a diverging conical or otherwise-shaped beam to simultaneously acquire an array of projections with dimensions along the direction of gantry rotation (that is, the O_{X} direction) and along the axial direction (that is, the O_{Z} direction). Resolution in such systems depends on the resolution of the array of x-ray detectors and on the rate of data acquisition, as specified for example in terms of the number of views per 360° rotation of the x-ray tube and the step size or helical pitch of the scan along the axial direction. Typically, the array of x-ray detectors is substantially isotropic in that the spacing of detectors along the O_{X} direction is about the same as the spacing of detectors along the O_{Z} direction.

To consider one example, for a detector array having an isotropic pitch of 0.08 cm in both the O_{X} and O_{Z} directions corresponding to a spatial frequency of 1/0.08 cm = 12.5 cm⁻¹, the maximum data sampling determined by Nyquist theorem is about one-half this value, or about 6.25 samples/cm. Data sampling in the O_{X} direction can be doubled using dual focal spot modulation in which the focal spot is spatially modulated in the O_{X} direction by an amount equivalent to a half-detector shift in the O_{X} direction. Data sampling in the O_{X} direction can also be doubled by combining opposing rays having a quarter-detector shift. By using both dual focal spot modulation and quarter detector shifting, a factor of four improvement in data sampling in the O_{X} direction is obtained. For the example isotropic array having isotropic 0.08 cm pitch, the data sampling in the O_{X} direction can be increased to 25 samples/cm by using both dual focal spot modulation and by combining offset opposing rays.

Obtaining a similar data sampling improvement in the O_{Z} direction using these techniques is difficult. There is generally no analog to the quarter-detector shift technique for the O_{Z} direction, and focal spot modulation in the O_{Z} direction is complicated by the x-ray tube anode geometry. For an isotropic x-ray detector array, employing both dual focal spot modulation and quarter-detector shifting in the O_{X} direction with no similar data sampling improvement technique applied in the O_{Z} direction results in highly anisotropic data sampling. For the example 0.08 cm isotropic radiation detector array, the data sampling in the O_{X} direction is 25 samples/cm while the data sampling in the O_{Z} direction is only 6.25 samples/cm, corresponding to about a 4:1 anisotropy in the acquired imaging data.

For clinical applications, an isotropic data sampling is usually advantageous. For example, data sampling of 25 samples/cm × 6.25 samples/cm is generally inferior to an equivalent isotopic data sampling of 12 samples/cm × 12 samples/cm. Moreover, an anisotropic data sampling tends to generate increased reconstructed image pixel noise.

A CT scanner system with staggered detector elements along the x axis or the z axis is disclosed in US 5,510,622. EP 0 543 626 A1 discloses a CT system featuring focal spot wobbling. DE 101 64233A1 discloses a CT system having detector modules.

The present invention contemplates an improved apparatus and method as defined in claims 1 and 3 and dependent claims 2 and 4 that overcomes the aforementioned limitations and others.

The invention may take form in various components and arrangements of components, and in various process operations and arrangements of process operations. The drawings are only for the purpose of illustrating preferred embodiments and are not to be construed as limiting the invention.
FIGURE 1 shows a diagrammatic representation of a computed tomography imaging system.
FIGURE 2 shows a diagrammatic representation of detector elements of a portion of the radiation detector array of FIGURE 1.
FIGURE 3 diagrammatically shows intersection points of projection samples with the meridian plane when using the detector array with detector elements staggered in the axial direction.
FIGURE 4A diagrammatically shows intersection points of projection samples with the meridian plane using the detector array with detector elements staggered in the axial direction and further employing dual focal spot modulation to produce samples shifted by a distance equivalent to a one-half detector spacing shift in the direction transverse to the axial direction.
FIGURE 4AA diagrammatically shows the dual focal spot modulation of FIGURE 4A viewed along the O_{Z} direction and transverse to the O_{U} direction. Four example projections are diagrammatically for each focal spot using solid arrows for the first focal spot and dashed arrows for the second focal spot.
FIGURE 4B diagrammatically shows intersection points of projection samples with the meridian plane using the detector array with detector elements staggered in the axial direction and employing dual focal spot modulation to produce samples shifted by a distance equivalent to a one-half detector spacing shift in the direction transverse to the axial direction, and further combining opposing projections separated by 180° plus an offset corresponding to one-quarter of the detector spacing in the direction transverse to the axial direction.
FIGURE 5A diagrammatically shows intersection points of projection samples with the meridian plane using the detector array with detector elements staggered in the axial direction and further employing dual focal spot modulation to produce samples shifted by a distance equivalent to one detector spacing in the direction transverse to the axial direction.
FIGURE 5B diagrammatically shows intersection points of projection samples with the meridian plane using the detector array with detector elements staggered in the axial direction and employing dual focal spot modulation to produce samples shifted by a distance equivalent to one detector spacing in the direction transverse to the axial direction, and further combining opposing projections separated by 180° plus an offset corresponding to one-half of the detector spacing in the direction transverse to the axial direction.
FIGURE 6 diagrammatically shows intersection points of projection samples with the meridian plane using the detector array with detector elements staggered in the axial direction and further employing dual focal spot modulation to produce samples shifted in the meridian plane by a distance equivalent to a detector shift of one-and-a-half times a detector spacing in the direction transverse to the axial direction.
FIGURE 7A diagrammatically shows only those projection samples of FIGURE 6 acquired with one linear array of detector elements.
FIGURE 7B diagrammatically shows only those projection samples of FIGURE 6 acquired with another linear array of detector elements.
FIGURE 8 diagrammatically shows intersection points of projection samples with the meridian plane using the detector array with detector elements staggered in the axial direction and further employing triple focal spot modulation with the three focal spots spaced apart to produce samples shifted in the meridian plane by a distance equivalent to shifts of two-thirds of a detector spacing in the direction transverse to the axial direction.
FIGURE 9A shows a diagrammatic representation of a detector having staggered detector modules, rather than staggered individual detector columns. In FIGURE 9, each detector module has four detector elements along the O_{U} direction.
FIGURE 9B diagrammatically shows intersection points of projection samples with the meridian plane when using the detector of FIGURE 9A with a dual focal spot modulation in which the focal spots are separated by a distance 4·**du.**

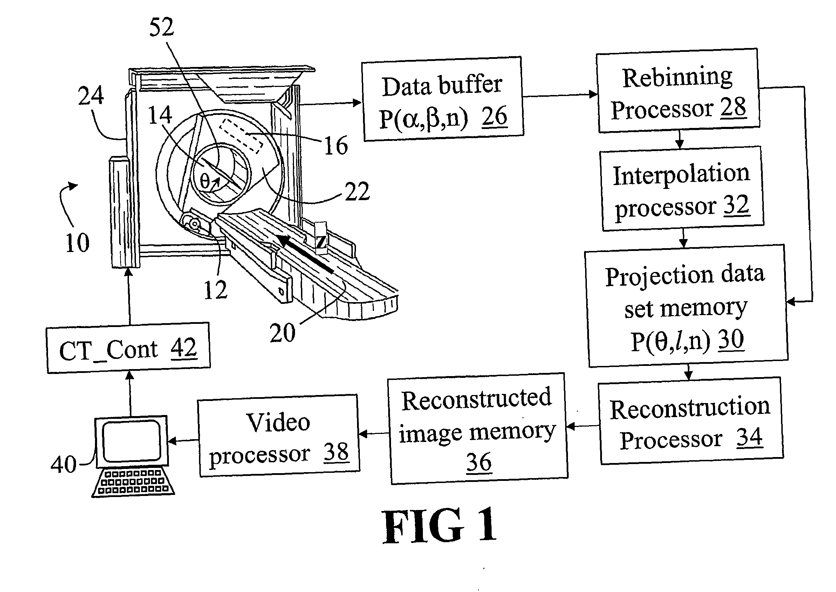

With reference to FIGURE 1, a computed tomography scanner **10** houses or supports a radiation source **12,** which in one embodiment is an x-ray source, that projects a radiation beam into an examination region **14** defined by the scanner **10.** After passing through the examination region **14,** the radiation beam is detected by a radiation detector array **16** (shown diagrammatically in phantom in FIGURE 1) arranged to detect the radiation beam after passing through the examination region **14.** In a typical x-ray tube embodiment of the radiation source **12,** the x-ray tube produces a diverging x-ray beam having a cone beam, wedge beam, or other beam geometry that expands as it passes through the examination region **14** to substantially fill the area of the radiation detector array **16.**

An imaging subject is placed on a couch **20** or other support that moves the imaging subject into the examination region **14.** The couch is linearly movable along an axial direction designated as a Z-direction in FIGURE 1. The radiation source **12** and the radiation detector **16** are oppositely mounted respective to the examination region **14** on a rotating gantry **22,** such that rotation of the gantry **22** effects revolving of the radiation source **12** about the examination region **14** to provide an angular range of views. The acquired data is referred to as projection data since each detector element detects a signal corresponding to an attenuation line integral taken along a line, narrow cone, or other substantially linear projection extending from the source to the detector element. The radiation detector 16 is shown mounted on the rotating gantry **22** in FIGURE 1; however, it is also contemplated to replace the x-ray detector array **16** by a band of x-ray detector elements mounted around a stationary gantry **24.**

In one embodiment, an axial projection data set is acquired with the rotating gantry **22** rotating while the couch **20** is stationary. The axial projection data set includes a plurality of axial slices corresponding to rows or columns of detector elements transverse to the axial or Z-direction. Optionally, additional axial slices are acquired by performing repeated axial scans and moving the couch **20** between each axial scan.

In another embodiment, a helical projection data set is acquired by rotating the gantry **22** simultaneously with continuous linear motion of the couch **20** to produce a helical trajectory of the radiation source **12** around the imaging subject disposed on the couch **20.** It will be appreciated that typically the same computed tomography scanner **10** can perform either axial or helical data acquisition depending upon whether the couch **20** is selected to move simultaneously with acquisition of imaging data.

In operation, some portion of the radiation passing along each projection is absorbed by the imaging subject to produce a generally spatially varying attenuation of the radiation. The detector elements of the radiation detector array **16** sample the radiation intensities across the radiation beam to generate radiation absorption projection data. As the gantry **22** rotates so that the radiation source **12** revolves around the examination region **14,** a plurality of angular views of projection data are acquired, collectively defining a projection data set that is stored in a buffer memory **26.**

For a source-focused acquisition geometry in a multi-slice scanner, readings of the attenuation line integrals or projections of the projection data set stored in the buffer memory **26** can be parameterized as P(α,β,n) where α is the source angle of the radiation source **12** determined by the position of the rotating gantry **22,** β is the angle within the fan (β∈ [-Φ/2, Φ/2] where Φ is the fan angle), and n is the detector row number in the O_{Z} direction. A rebinning processor **28** rebins the projection data into a parallel, non-equidistant raster of canonic trans-axial coordinates. This rebinning can be expressed as P(α,β,n) → P(θ,*l*,n) where θ parameterizes the projection number that is composed of parallel readings parameterized by 1 which specifies the distance between a reading and the isocenter, and n is the detector row number.

The rebinned parallel ray projection data set P(θ,*l*,n) is stored in a projection data set memory **30.** Optionally, the projection data is interpolated by a rebinning processor **32** into equidistant coordinates or into other desired coordinates spacings before storing the projection data P(θ,*l*,n) in the projection data set memory **30.** A reconstruction processor **34** applies filtered backprojection or another image reconstruction technique to reconstruct the projection data set into one or more reconstructed images that are stored in a reconstructed image memory **36.** The reconstructed images are processed by a video processor **38** and displayed on a user interface **40** or is otherwise processed or utilized. In one embodiment, the user interface **40** also enables a radiologist, technician, or other operator to interface with a computed tomography scanner controller **42** to implement a selected axial, helical, or other computed tomography imaging session.

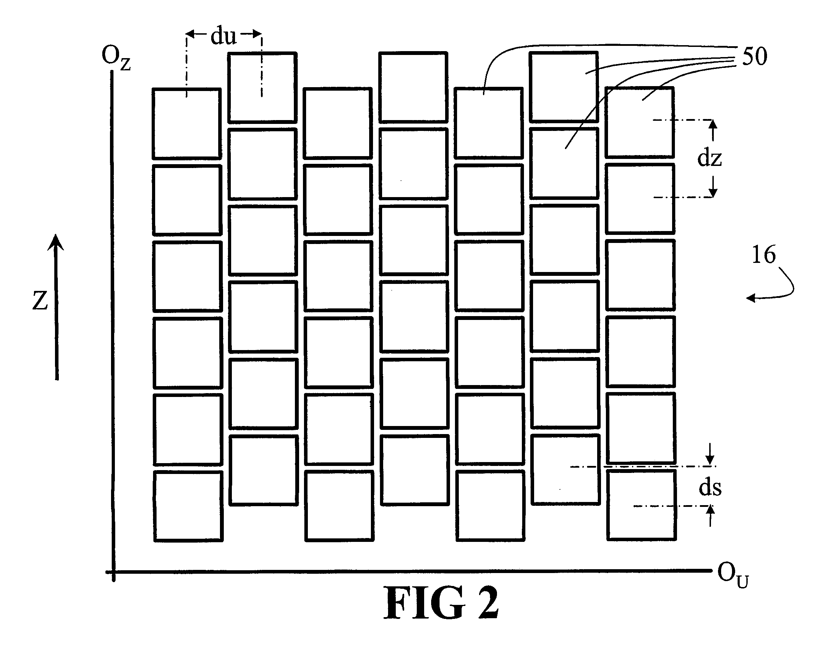

With reference to FIGURE 2 and as example not part of the invention, a portion of the radiation detector **16** is shown. The radiation detector **16** is described with reference to a detector coordinate directions (O_{U}, O_{Z}), in which the O_{Z} direction is parallel to the axial or Z-direction of FIGURE 1, and O_{U} is transverse to the axial or Z-direction. The radiation detector array **16** includes rows of detector elements **50** having a spacing dz along the O_{Z} direction. Each row of detector elements **50** can be considered as a linear detector array of detector elements **50** spaced apart by the detector spacing **dz.** The linear detector arrays are spaced apart transverse to the axial or O_{Z} direction by a detector spacing **du.** That is, the detector elements **50** are spaced apart along the O_{U} or circumferential direction by the detector spacing **du.** The detector spacings **du, dz** transverse to and along the axial Z-direction, respectively, are approximately equal in the detector **16** shown in FIGURE 2, providing an isotropic array. However, it is also contemplated to use different detector spacings along the O_{U} and O_{Z} directions, thus producing an anisotropic detector array.

The detectors **50** are staggered along the axial or Z-direction by a staggering fraction **ds** of the spacing **dz** along the axial or O_{Z} direction. That is, adjacent linear detector arrays have detector elements spatially offset along the axial or Z-direction by the staggering fraction **ds.** In FIGURE 2, the staggering fraction **ds** is one-half of the spacing **dz** along the axial or O_{Z} direction; however, other staggering fractions are contemplated. The one-half detector spacing staggering advantageously provides a doubled sampling rate in the axial or O_{Z} direction. Analogously, staggering by one-third of the detector spacing **dz** triples the sampling rate in the O_{Z} direction, and so forth for other staggering intervals. The staggering of the detector elements **50** along the axial or Z-direction does not increase the sampling rate in the O_{U} direction which is transverse to the axial direction.

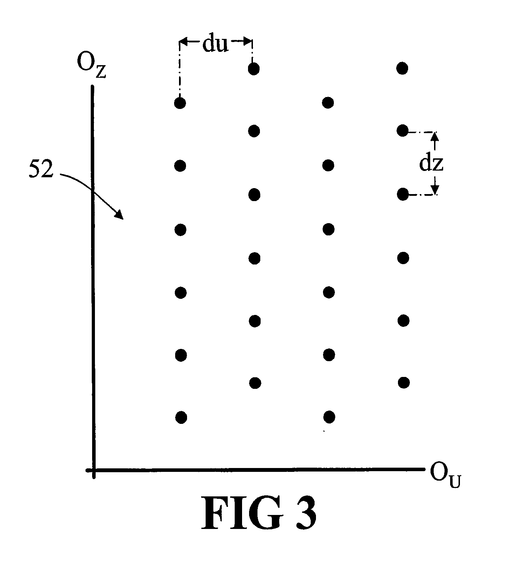

FIGURE 3 plots filled circles representing points of intersection between projections having a given projection parameter θ and a meridian plane **52.** The meridian plane **52** is defined as the plane containing the axis of rotation of the gantry **22** that is perpendicular to the projections of projection parameter θ. One example meridian plane **52** for an example projection parameter θ is indicated diagrammatically in FIGURE 1. As can be seen in FIGURE 3, the staggering of detector elements in the O_{Z} direction produces a doubling of sampling rate the O_{Z} direction. For isotropic detector spacings **du, dz,** the sampling using the staggered detector array **16** is anisotropic with a 1:2 ratio of sampling rates in the O_{U} and O_{Z} directions, respectively.

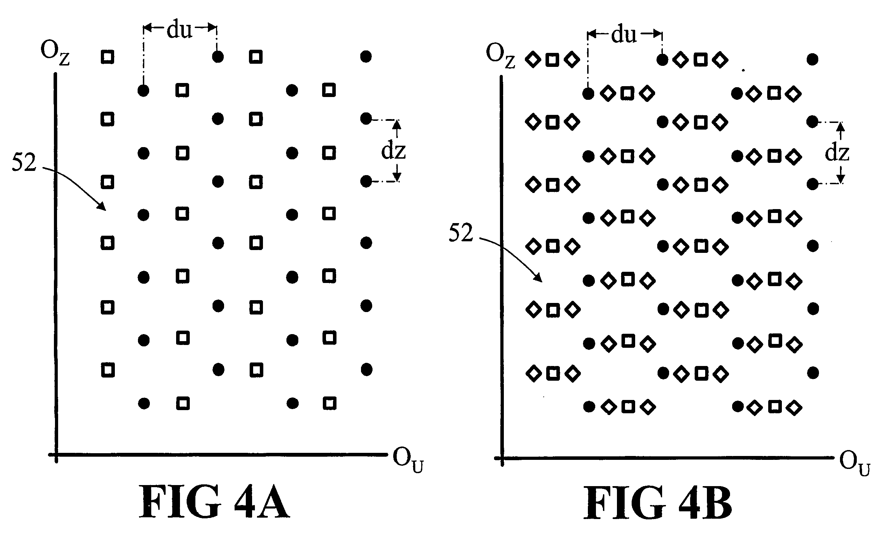

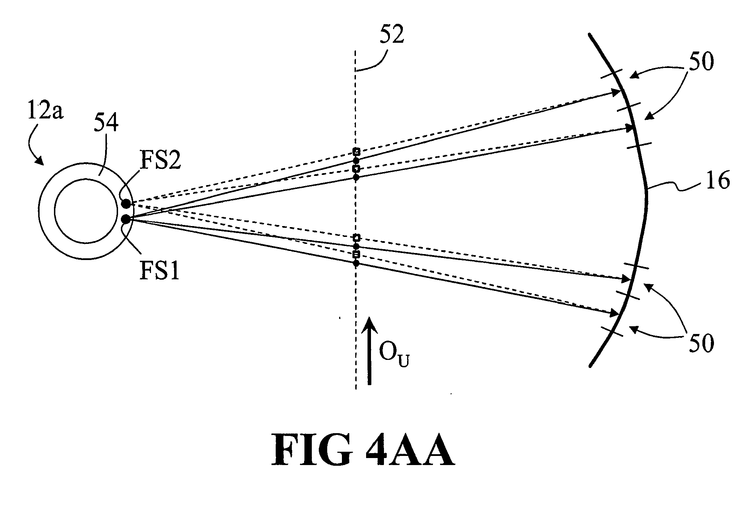

With reference to FIGURES 4A and 4AA, in one example not part of the invention a higher sampling rate in the O_{U} direction is obtained using dual focal spot modulation of the radiation source **12** in the O_{U} direction. The focal spot is shifted between two positions **FS1** and **FS2** (labeled in FIGURE 4AA) on a beveled surface **54** of an anode **12a** of the radiation source **12.** The separation of the focal spots **FS1, FS2** at the anode **12a** is selected to shift the projections at the meridian plane **52** by a distance equivalent to shifting the detectors **50** by a distance of one-half of the detector spacing **du.** In FIGURES 4A and 4AA, filled circles on the meridian plane **52** indicate samples acquired using the focal spot **FS1,** and open squares on the meridian plane **52** indicate samples acquired using the focal spot **FS2.** FIGURE 4AA does not take into account gantry rotation. If gantry rotation is included, the focal spot shift can be selected to rotate the shifted spot back to the original focal spot position for a particular sampling geometry. It is seen that the sampling rate at the meridian plane **52** is doubled in the O_{U} direction by the dual focal spot modulation so that the O_{U} sampling rate matches the O_{Z} sampling rate. For isotropic detector spacings **du, dz,** the sampling illustrated in FIGURES 4A and 4AA is isotropic with a 1:1 O_{U}:O_{Z} sampling ratio. However, as seen in FIGURE 4A the samples are not arranged in a rectangular grid on the meridian plane **52.**

With reference to FIGURE 4B, a still higher sampling rate in the O_{U} direction is optionally achieved by using the dual focal spot shifting with a shift of one-half of the detector spacing **du** as described with reference to FIGURES 4A and 4AA, and by additionally combining opposing projections acquired at 180° rotated from the initial projections. More particularly, the opposing projections are located at an angle which is 180° plus an offset rotated from the initial projections, where the offset is elected to provide a shift in the meridian plane corresponding to a shift of plus or minus one-fourth of the detector spacing **du.** These opposing projections are represented in FIGURE 4B by open diamonds. For isotropic detector spacings **du, dz**, the sampling at the meridian plane **52** in FIGURE 4B is anisotropic with a 2:1 O_{U}:O_{Z} sampling ratio. The samples are not arranged in a rectangular grid.

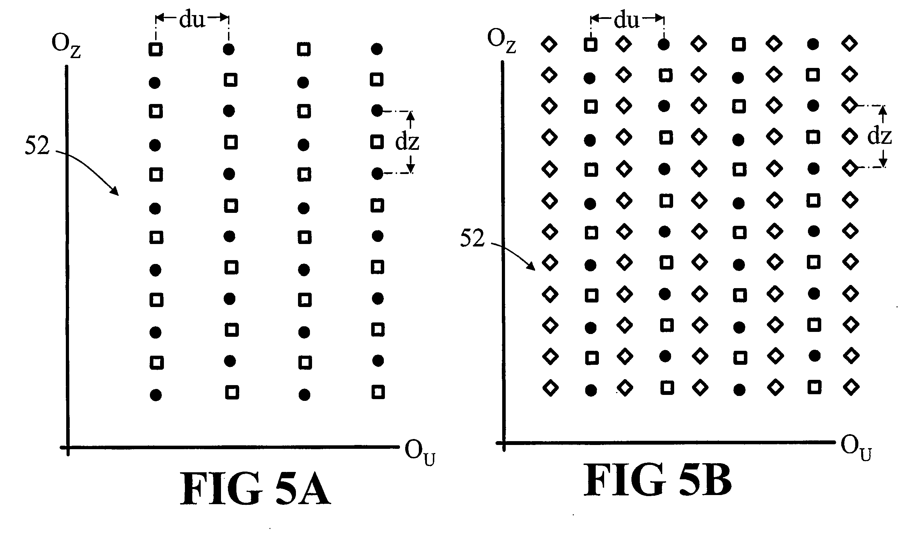

With reference to FIGURE 5A, in another example not part of the invention dual focal spot modulation in the O_{U} direction of the radiation beam produced by the radiation source **12** is used to produce an anisotropic sampling that is arranged in a Cartesian grid. The focal spot shift at the anode is selected to shift the projections at the meridian plane **52** by a distance equivalent to shifting the detectors **50** by a distance of one detector spacing **du.** In FIGURE 5A, filled circles indicate samples acquired using a first focal spot position, and open squares indicate samples acquired using a second focal spot position that produces a sample shift at the meridian plane **52** equivalent to a one detector spacing **du** shift of the detectors **50.** The sampling rate is unchanged in the O_{U} direction by the dual focal spot modulation equivalent to one detector spacing **du**. However, the second focal spot samples (indicated by the open squares) combined with the first focal spot samples (indicated by the filled circles) define a rectangular grid of samples in the meridian plane **52.** For isotropic detector spacings **du, dz,** the sampling in FIGURE 5A is anisotropic with a 1:2 O_{U}:O_{Z} sampling ratio.

With reference to FIGURE 5B, a doubled sampling rate in the O_{U} direction is optionally achieved by using the dual focal spot shifting illustrated in FIGURE 5A, and by additionally combining opposing projections acquired at an angle that is 180° plus an offset rotated from the initial projections. The offset is selected to provide a sample shift in the meridian plane **52** equivalent to a plus or minus one-half detector spacing **du** shift of the detectors **50.** These opposing projections are represented in FIGURE 5B by open diamonds. For isotropic detector spacings **du, dz,** the sampling in at the meridian plane **52** shown in FIGURE 5B is isotropic with a 1:1 O_{U}:O_{Z} sampling ratio, and the samples are arranged in a rectangular grid.

The approaches of FIGURES 4B and 5B combine approximately 180° opposing projections into a single projection data set, and are readily performed for axial scans in which the subject support **20** remains stationary during data acquisition. Stepwise linear movement of the couch **20** is optionally performed between axial scans to acquire a projection data set covering a volume along the axial or Z-direction that is larger than the span of the detector array **16** along the axial or Z-direction.

For helical computed tomography imaging, the opposing rays are separated by a distance related to about one-half of the helical pitch due to linear motion of the subject support **20** along the Z-direction during the 180° revolving of the radiation source **12.** Considering axial scans and helical scans with medium cone angles, it has been shown that using three-dimensional back projection methods, it is possible to increase the data sampling along the O_{U} direction by combining 180° opposing projections without introducing substantial image artifacts. See Gilad Shechter, "High-resolution images of cone beam collimated CT scans," WO03/094115A1 (international application no. PCT/IL02/00355).

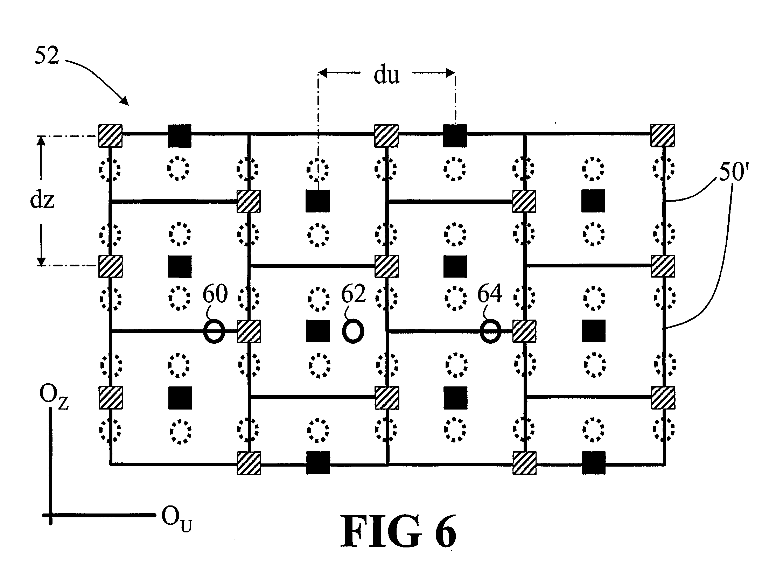

With reference to FIGURE 6, an approach not part of the invention for obtaining rectangular sampling in the meridian plane **52** at improved sampling rates without combining opposing projections is described. FIGURE 6 shows points of intersection between projections having a common projection parameter θ and the meridian plane **52** corresponding to that projection parameter θ. In FIGURE 6, filled squares indicate samples acquired at a first focal spot position, while hatched squares indicate samples acquired using a second focal spot that produces a shift corresponding to one-and-a-half times the detector spacing **du** at the meridian plane **52.** Also drawn in FIGURE 6 are images **50'** of the edges of detectors **50** projected onto the meridian plane **52** along projection lines corresponding to the first focal spot. The detector edge representations **50'** illustrate the equivalent one-and-a-half detector spacing shift between samples acquired using the first and second focal spots.

Data acquired using the two focal spots, corresponding to the combination of filled and hatched squares, represents intersections of the projection samples with the meridian plane **52.** This data has doubled sampling rates in both the O_{U} and the O_{Z} directions. However, the data does not correspond to sampling arranged in an isotropic rectangular grid. Optionally, the projection data is interpolated by the interpolation processor **32** to produce interpolated projection samples represented by dotted open circles in FIGURE 6. The interpolated samples are also seen to have doubled sampling rates in both the O_{U} and the O_{Z} directions. Additionally, however, the interpolated samples are arranged in an isotropic rectangular grid. The interpolation processor **32** can perform the interpolation using a two-dimensional filter having dimensions in both the O_{U} and O_{Z} directions and including, for example, eight coefficients. Alternatively, one-dimensional interpolation in the Z-direction can be employed, or another interpolation technique can be used.

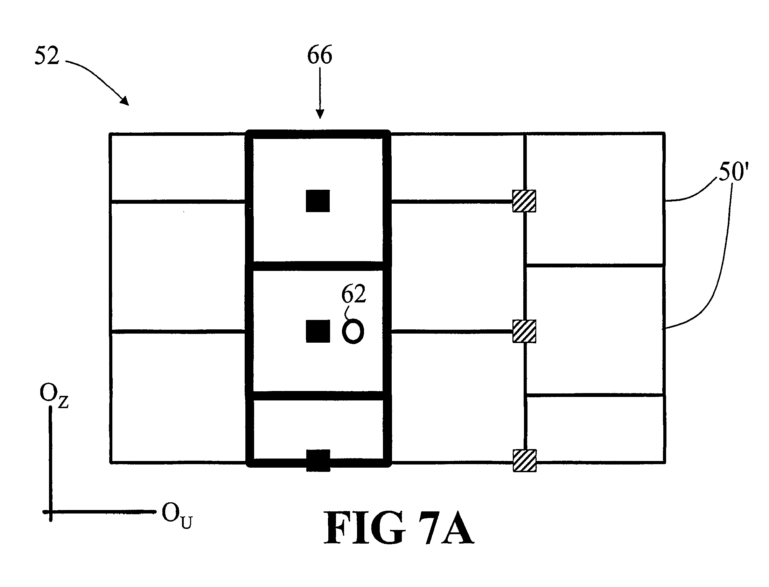

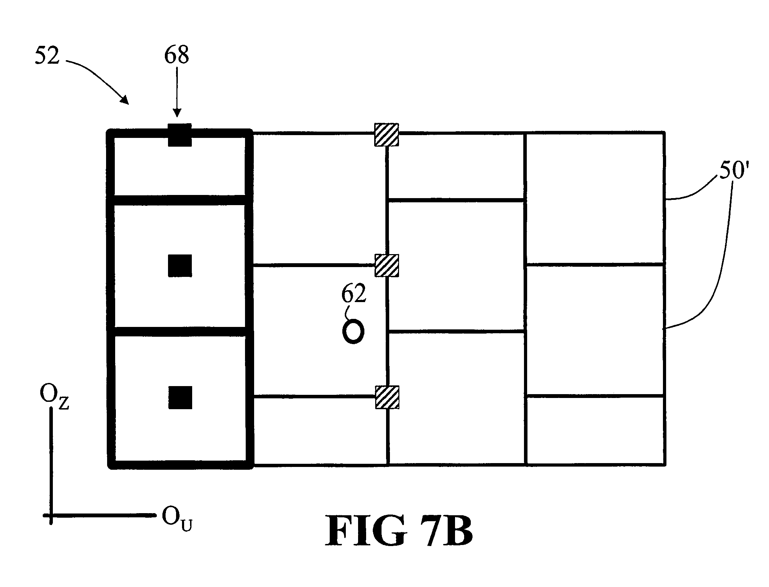

With continuing reference to FIGURE 6 and with further reference to FIGURES 7A and 7B, correction of ring artifacts produced by miscalibrated or otherwise imperfect detectors is optionally performed using redundantly interpolated adjacent detector row positions. In FIGURE 6, example ring correction points **60, 62, 64** for three rows are indicated by open circles. FIGURE 7A shows only those projection samples acquired using a linear detectors array indicated by bold detector edge representations **66** in FIGURE 7A. Similarly, FIGURE 7B shows only those projection samples acquired using an adjacent linear detectors array indicated by bold detector edge representations **68** in FIGURE 7B. In one example approach to ring correction, the samples of FIGURE 7A are combined to produce a first interpolated value for the ring correction point **62.** The samples of FIGURE 7B are similarly combined to produce a second interpolated value for the ring correction point **62.** Differences between the first and second sample values are indicative of differences between the linear detectors arrays **66, 68** caused by a miscalibrated or otherwise problematic detector in one of the linear detectors arrays **66, 68.** One contemplated ring correction filter employs view-filtered differences of interpolated values for the same position in the meridian plane **52** obtained using different linear detector arrays.

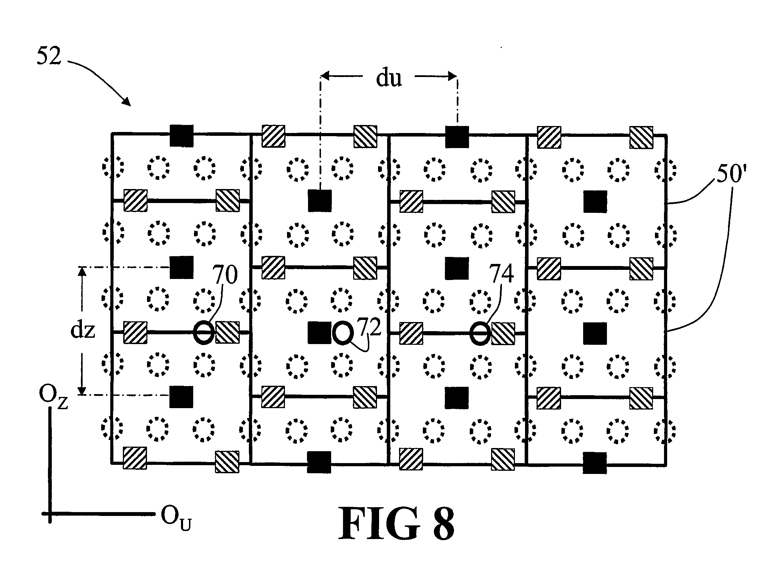

With reference to FIGURE 8, an approach not part of the invention for obtaining rectangular sampling at improved sampling rates uses triple focal spot modulation. In FIGURE 8, filled squares indicate samples acquired at a first focal spot position. Hatched squares indicate samples acquired using a second focal spot which produces a shift of two-thirds of the detector spacing **du** at the meridian plane **52.** Oppositely hatched squares indicate samples acquired using a third focal spot which produces a shift of four-thirds of the detector spacing **du** at the meridian plane **52** relative to samples produced by the first focal spot (or, equivalently, a shift of two-thirds of the detector spacing **du** relative to samples produced by the second focal spot). It will be appreciated that projections using the first, second, and third focal spots need not be acquired in that order; rather, for example, the data using the second focal spot can be acquired first, followed by data acquisition using the third focal spot, followed thereafter by data acquisition using the first focal spot. For convenience, also illustrated in FIGURE 8 are projections **50'** of the edges of detectors **50** onto the meridian plane **52** along projection lines corresponding to the first focal spot. The detector projections **50'** illustrate the two-thirds detector spacings between samples acquired using the first, second, and focal spots.

Using the triple focal spot sampling, the sampling in the O_{U} direction is tripled, while the sampling in the O_{Z} direction is doubled. However, the data does not correspond to sampling arranged in a rectangular grid in the meridian plane **52.** Optionally, the data is interpolated by the interpolation processor **32** to produce interpolated samples represented by dotted open circles in FIGURE 8. The interpolated samples are also seen to have a tripled sampling rate in the O_{U} direction and a doubled sampling rate in the O_{z} direction. Additionally, however, the interpolated samples are arranged in a rectangular grid.

Moreover, correction of ring artifacts produced by miscalibrated or otherwise imperfect detectors is optionally performed using redundantly interpolated adjacent detector row positions. In FIGURE 7, example ring correction points **70, 72, 74** for three rows are indicated by open circles. A suitable ring correction filter employs view-filtered differences of interpolated values for the same position in the meridian plane **52** obtained using different linear detector arrays.

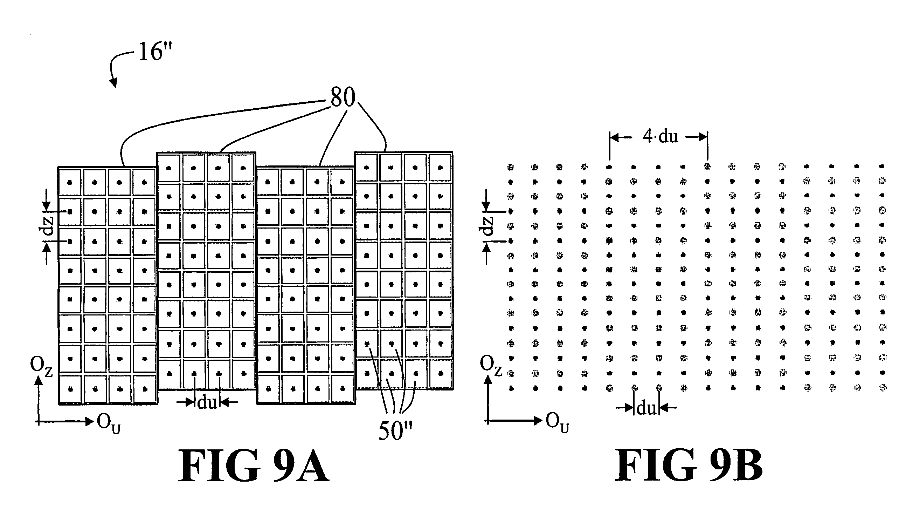

With reference to FIGURES 9A and 9B, in some embodiments according to the invention the staggering in the z-direction is with respect to detector modules rather than with respect to individual detector elements or individual linear detector columns. In example FIGURE 9A, a detector array **16"** includes detector elements **50"** arranged into two-dimensional detector modules **80.** Each detector module **80** includes four columns of detector elements **50"** spaced apart by spacing **du** in the O_{U} direction. The detector modules **80** are spaced apart by a spacing of 4·**du** in the O_{U} direction, so that the spacing **du** of the detector elements **50"** in the O_{U} direction is maintained across the boundaries of the detector modules **80.**

Within each detector module **80,** the detector elements **50"** are spaced apart by spacing dz in the O_{Z} direction (that is, the axial or Z direction). There is no staggering of the detector elements **50"** in the O_{Z} direction within each detector module **80,** and so the detector elements 50" within each detector module **80** define a regular two-dimensional array. The staggering of detector elements is produced by staggering the detector modules **80** by a spacing **dz**/2 in the O_{Z} direction, as shown in FIGURE 9A. The effect for the example modules **80** having four detector columns per module is to stagger every contiguous group of four detector elements **50"** along the O_{U} direction, rather than staggering every detector element as is shown in, for example, FIGURE 2.

This module-based staggering approach can have manufacturing advantages, since the radiation detectors are typically manufactured in two-dimensional detector modules having several columns of detectors spaced apart along the O_{U} direction.

With reference to FIGURE 9B, in order to achieve interleaved sampling in conjunction with the detector array **16",** the two focal spots of the dual focal spot modulation should be separated by a distance 4·**du** in the O_{U} direction so as to accommodate the staggering of groups of four detector elements **50"** along the O_{U} direction. While the illustrated detector modules **80** have four detector elements **50"** along the O_{U} direction, detector modules of other sizes can be similarly staggered, and the dual focal spot modulation should be similarly adjusted. If, for example, each detector module has only two detector elements along the O_{U} direction, then the focal spots should be separated by a distance of 2 **du** in the O_{U} direction.

The invention has been described with reference to the preferred embodiments. Obviously, modifications and alterations will occur to others upon reading and understanding the preceding detailed description. It is intended that the invention be construed as including all such modifications and alterations insofar as they come within the scope of the appended claims or the equivalents thereof.

## Claims

1. A computed tomography imaging apparatus comprising:
a support for moving an imaging subject and being linearly movable along an axial direction (z);
a radiation detector (16"), wherein the radiation detector (16") comprises a plurality of two-dimensional detector modules (80) each including a plurality of linear detector arrays, each linear detector array including detector elements (50") spaced apart by a detector spacing (dz) along the axial direction (Z), the linear detector arrays being spaced apart transverse to the axial direction (Z) by a detector spacing (du), neighboring linear detector arrays not being staggered along the axial direction (Z), neighboring detector modules (80) being staggered along the axial direction (Z) by one-half of the spacing (dz) of detector elements;
a radiation source (12) providing focal spot modulation that increases a sampling rate transverse to the axial direction (Z), wherein the focal spot modulation produces two projections spaced apart transverse to the axial direction (z) by a distance corresponding to the detector spacing (du) times a number of detector elements in each of the detector modules (80), the detector modules each being a contiguous group of detector elements along the direction transverse to the axial direction (Z), such as to achieve interleaved sampling; and
a reconstruction processor to reconstruct a projection data set acquired with the projections into at least one image.

2. The computed tomography imaging apparatus of claim 1, wherein each detector module (80) includes four linear detector arrays.

3. A computed tomography imaging method comprising:
providing a support for moving an imaging subject and being linearly movable along an axial direction (z); the staggering detectors (50") of a detector array (16") in the axial direction (Z) by a selected non-zero staggering fraction (ds) of a detector spacing (dz) along the axial direction (Z) and;
modulating a radiation source (12) irradiating the detector array (16") between at least two focal spot positions spaced apart transverse to the axial direction (Z),
wherein the staggering of detectors (50") includes staggering along the axial direction (Z) neighboring contiguous groups of detector elements (50"), each contiguous group including a plurality of detector elements spaced apart by a detector spacing (du) along the direction transverse to the axial direction (Z), such as to achieve interleaved sampling;
wherein the at least two focal spot positions produce two projections spaced apart transverse to the axial direction (Z) by a distance corresponding to the detector spacing (du) times a number of detector elements in each contiguous group of detector elements along the direction transverse to the axial direction (Z); the method further comprising
reconstructing a projection data set acquired with the projections into at least one image.

4. A computed tomography apparatus for performing the method of claim 3.

## Patentansprüche

1. Computertomographie-Bildgebungsgerät, das Folgendes umfasst:
eine Auflage zum Bewegen eines Bildgebungsobjekts, die linear entlang einer axialen Richtung (Z) verschiebbar ist;
einen Strahlungsdetektor (16"), wobei der Strahlungsdetektor (16") eine Vielzahl von zweidimensionalen Detektormodulen (80) umfasst, die jeweils eine Vielzahl linearer Detektorarrays enthalten, wobei jedes lineare Detektorarray Detektorelemente (50") enthält, die in der axialen Richtung (Z) um einen Detektorabstand (dz) voneinander getrennt sind, wobei die linearen Detektorarrays quer zu der axialen Richtung (Z) durch einen Detektorabstand (du) voneinander getrennt sind, wobei benachbarte lineare Detektorarrays nicht in axialen Richtung (Z) gestaffelt sind, benachbarte Detektorarrays (80) in axialer Richtung (Z) um die Hälfe des Abstands (dz) der Detektorelemente gestaffelt sind;
eine Strahlungsquelle (12) zur Lieferung der Brennfleckmodulation, die eine Abtastrate quer zur axialen Richtung (Z) steigert, wobei die Brennfleckmodulation zwei Projektionen erzeugt, die quer zur axialen Richtung (Z) um einen Abstand voneinander getrennt sind, der dem Detektorabstand (du) mal einer Anzahl von Detektorelementen in jedem der Detektormodule (80), wobei die Detektormodule jeweils eine zusammenhängende Gruppe von Detektorelementen sind, in der Richtung quer zur axialen Richtung (Z) entspricht, um eine verschachtelte Abtastung zu erreichen; und
einen Rekonstruktionsprozessor zum Rekonstruieren eines mit den Projektionen erfassten Projektionsdatensatzes zu mindestens einem Bild.

2. Computertomographie-Bildgebungsgerät nach Anspruch 1, wobei jedes Detektormodul (1) vier lineare Detektorarrays umfasst.

3. Computertomographie-Bildgebungsverfahren, das Folgendes umfasst:
Liefern der Staffelung der Detektoren (50") eines Detektorarrays (16") in axialer Richtung (Z) durch eine ausgewählte Staffelungsfunktion (ds) ungleich Null eines Detektorabstands (dz) in axialer Richtung (Z); und
Modulieren einer das Detektorarray (16") beleuchtenden Strahlungsquelle (12) zwischen mindestens zwei Brennfleckpositionen, die um einen Abstand quer zur axialen Richtung (Z) getrennt sind,
wobei das Staffeln von Detektoren (50") das Staffeln von benachbarten zusammenhängenden Gruppen von Detektorelementen (50") in axialer Richtung (Z) umfasst, wobei jede zusammenhängende Gruppe eine Vielzahl von Detektorelementen umfasst, die um einen Detektorabstand (du) in der Richtung quer zur axialen Richtung (Z) voneinander getrennt sind, um eine verschachtelte Abtastung zu erreichen,
wobei die mindestens zwei Brennfleckpositionen zwei Projektionen erzeugen, die quer zur axialen Richtung (Z) um einen Abstand voneinander getrennt sind, der dem Detektorabstand (du) mal einer Anzahl von Detektorelementen in jeder zusammenhängenden Gruppe von Detektorelementen in der Richtung quer zur axialen Richtung (Z) entspricht; wobei das Verfahren weiterhin Folgendes umfasst:
Rekonstruieren eines mit den Projektionen erfassten Projektionsdatensatzes zu mindestens einem Bild.

4. Computertomographie-Gerät zur Durchführung des Verfahrens nach Anspruch 3.

## Revendications

1. Appareil d'imagerie par tomographie assistée par ordinateur comprenant :
un support pour déplacer un objet d'imagerie et étant linéairement mobile le long d'une direction axiale (Z) ;
un détecteur de rayonnement (16") dans lequel le détecteur de rayonnement (16") comprend une pluralité de modules de détection bidimensionnels (80), chacun comprenant une pluralité de réseaux de détection linéaires, chaque réseau de détection linéaire comprenant des éléments de détection (50") qui sont espacés l'un de l'autre par un espacement de détection (dz) le long de la direction axiale (Z), les réseaux de détection linéaires étant espacés l'un de l'autre transversalement à la direction axiale (Z) par un espacement de détection (du), les réseaux de détection linéaires voisins n'étant pas échelonnés le long de la direction axiale (Z), les modules de détection voisins (80) étant échelonnés le long de la direction axiale (Z) par une moitié de l'espacement (dz) des éléments de détection ;
une source de rayonnement (12) fournissant une modulation de spot focal qui augmente un taux d'échantillonnage transversalement à la direction axiale (Z) où la modulation de spot focal produit deux projections qui sont espacées l'une de l'autre transversalement à la direction axiale (Z) par une distance correspondant à des fois d'espacement de détection (du) d'un certain nombre d'éléments de détection dans chacun des modules de détection (80), les modules de détection étant chacun un groupe contigu d'éléments de détection le long de la direction qui est transversale à la direction axiale (Z) de manière à réaliser un échantillonnage entrelacé ; et
un processeur de reconstruction pour reconstruire un ensemble de données de projection, qui est acquis avec les projections, en au moins une image.

2. Appareil d'imagerie par tomographie assistée par ordinateur selon la revendication 1, dans lequel chaque module de détection (80) comprend quatre réseaux de détection linéaires.

3. Procédé d'imagerie par tomographie assistée par ordinateur comprenant les étapes suivantes consistant à :
fournir un support pour déplacer un objet d'imagerie et étant linéairement mobile le long d'une direction axiale (Z) ;
échelonner les détecteurs (50") d'un réseau de détection (16") dans la direction axiale (Z) par une fraction sélectionnée d'échelonnement non-zéro (ds) d'un espacement de détection (dz) le long de la direction axiale (Z) ; et
moduler une source de rayonnement (12) rayonnant le réseau de détecteurs (16") entre au moins deux positions de spot focal qui sont espacées l'une de l'autre transversalement à la direction Axiale (Z),
dans lequel l'échelonnement des détecteurs (50") comprend l'échelonnement le long de la direction axiale (Z) avoisinant les groupes contigus d'éléments de détection (50"), chaque groupe contigu comprenant une pluralité d'éléments de détection qui sont espacés l'un de l'autre par un espacement de détection (du) le long de la direction qui est transversale à la direction axiale (Z) de manière à réaliser un échantillonnage entrelacé ;
dans lequel les au moins deux positions de spot focal produisent deux projections qui sont espacées l'une de l'autre transversalement à la direction axiale (Z) par une distance correspondant à des fois d'espacement de détection (du) d'un certain nombre d'éléments de détection dans chaque groupe contigu d'éléments de détection le long de la direction qui est transversale à la direction axiale (Z) ; le procédé comprenant encore l'étape suivante consistant à :
reconstruire un ensemble de données de projection, qui est acquis avec les projections, en au moins une image.

4. Appareil d'imagerie par tomographie assistée par ordinateur pour mettre en oeuvre le procédé de la revendication 3.
